# EUROPÄISCHE PATENTSCHRIFT

(11) **EP 0 520 264 B1**
(45) Veröffentlichungstag und Bekanntmachung des Hinweises auf die Patenterteilung: **29.03.1995**
(21) Anmeldenummer: 92109975.0
(22) Anmeldetag: 13.06.1992
(51) Int. Cl.: C07C 69/38, C07C 67/46

(54) **Verfahren zur Herstellung von Malonsäuredialkylestern**
Process for the preparation of malonic acid dialkyle esters
Procédé de préparation de diesters alkyliques d'acide malonique

(30) Priorität: 22.06.1991 DE 4120721
(43) Veröffentlichungstag der Anmeldung: 30.12.1992
(73) Patentinhaber: BASF Aktiengesellschaft, 67063 Ludwigshafen (DE)
(72) Erfinder: Schuster, Ludwig, Dr., W-6703 Limburgerhof (DE); Halbritter, Klaus, Dr., W-6900 Heidelberg (DE)

(56) Entgegenhaltungen:
- US-A- 4 251 447
- US-A- 4 360 691

## Beschreibung

Die Erfindung betrifft ein Verfahren zur Herstellung von Malonsäuredialkylestern durch Ozonolyse von Diketen und anschließender katalytischer Hydrierung sowie Veresterung der Reaktionsprodukte.

Es ist bekannt, Malonsäureanhydrid und substituierte Malonsäureanhydride durch Ozonolyse des Enol-Lakton-Dimeres des Ketens, des sog. Diketens, herzustellen. Die dabei entstehenden Malonsäureanhydride können mit Wasser zur entsprechenden Säure hydrolysiert werden, mit Alkoholen zu den entsprechenden Monoestern umgesetzt werden oder mit einem Amin in das Monoamid übergeführt werden (U.S. 4 360 691 und U.S. 4 251 447). Dies ist eine sehr leicht verlaufende Reaktion, charakteristisch für den Vierring des Malonsäureanhydrides. Aus diesen Literaturstellen geht jedoch hervor, daß man viel schärfere Bedingungen anwenden muß, um den Monoester oder das Monoamid in die entsprechenden Diester, Diamide oder Esteramide überzuführen, beispielsweise die Behandlung des Monoesters oder Monoamides mit Thionylchlorid, gefolgt von der Umsetzung der entstehenden Esterchloride oder Amid-Chlorid-Verbindung mit weiterem Alkohol oder Amin. Bei dieser Reaktionsweise wurden gemäß den übereinstimmenden Beispielen 2 der beiden US-Patente nur 76 % des erwarteten Malonsäurehalbesters isoliert. Auch mit unsubstituiertem Diketen werden nach den Beispielen 3 nur 76 % Malonsäurehalbester erhalten.

Die Aufarbeitung des Reaktionsgemisches ist wegen der auftretenden Peroxiverbindungen schwierig. Die Reaktionsprodukte müssen durch alkalische Extraktion aus dem Reaktionsgemisch isoliert und aus diesem Extrakt durch mehrfaches Waschen mit Ether nach dem Ansäuern gewonnen werden. Zur Gewinnung z.B. der Diester sind, wie oben erwähnt, weitere Reaktionsschritte notwendig.

Die wesentlichen Reaktionsprodukte sind jedoch die Malonsäurediester, da diese wirtschaftlich bedeutungsvoll sind und z.B. zur Barbitursäuresynthese eingesetzt werden können.

Der Erfindung lag daher die Aufgabe zugrunde, ein Verfahren zur Verfügung zu stellen, durch das die Malonsäuredialkylester in hoher Ausbeute einfach und möglichst ohne das Auftreten von nicht verwertbaren Nebenprodukten herstellbar sind.

Es wurde nun gefunden, daß diese Aufgabe durch ein Verfahren gelöst werden kann, bei dem Malonsäuredialkylester und daneben Dialkylacetale und Alkylformiate entstehen, das dadurch gekennzeichnet, daß man
a) Diketen in einem C₁-C₆-Alkanol ozonisiert,
b) das Reaktionsgemisch anschließend unter Bildung der Alkylformiate katalytisch hydriert und
c) dann unter Zugabe katalytischer Säuremengen zur Bildung der Dialkylacetale und der Malonsäuredialkylester erwärmt.

Mit dieser Reaktionsfolge lassen sich die Malonsäuredialkylester in hohen Ausbeuten herstellen, obwohl die thermische Instabilität der Malonsäure und des Malonsäureanhydrids bekannt ist.

Die erfindungsgemäße Reaktionsfolge läßt sich schematisch wie folgt darstellen: 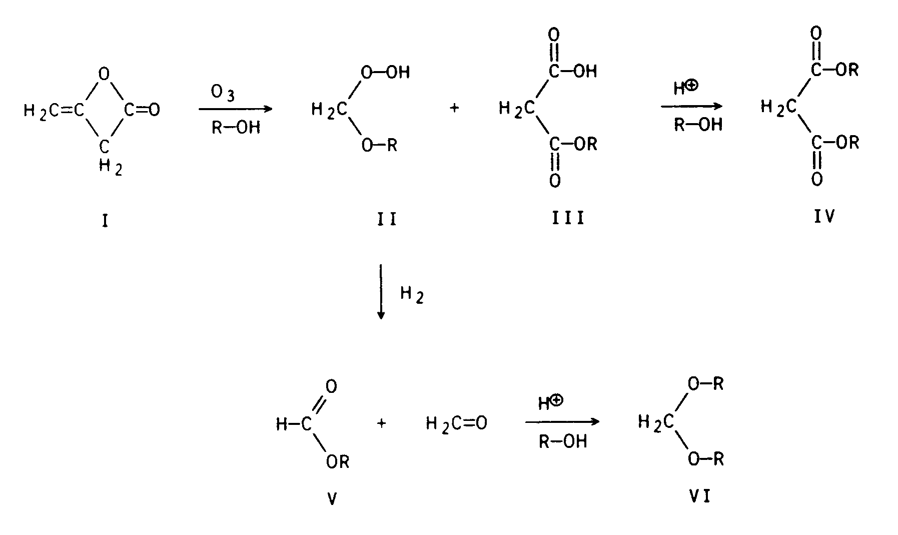
Das Diketen (I) wird in einem C₁-C₆-Alkanol ozonisiert, wobei zunächst ein Hydroperoxihemiacetal (II) und ein Malonsäurehalbester (III) entstehen. Sei der anschließenden katalytischen Hydrierung entsteht aus (II) ein Alkylformiat (V) und Formaldehyd. Unter Zugabe von katalytischen Mengen Säure, z.B. unter Bedingungen einer azeotropen Veresterung, entsteht aus dem Formaldehyd das Dialkylacetal (VI), auch als Dialkylformal zu bezeichnen, und aus dem Halbester (III) der erwünschte Malonsäuredialkylester (IV).

Die gesamte Reaktion kann als "Eintopfreaktion" ablaufen. Als Alkohole sind C₁-C₄-Alkanole, insbesondere Methanol und Ethanol bevorzugt.

Die Reaktion kann sowohl kontinuierlich als auch diskontinuierlich geführt werden.

Als besonderer Vorteil ergibt sich bei dem erfindungsgemäßen Verfahren, daß Nebenprodukte entstehen, die ebenfalls verwertbar sind, und das Auftreten von störenden Reaktionsprodukten weitgehend vermieden wird. So entsteht bei der Reaktion des als Zwischenprodukt auftretenden Hydroperoxihemiacetals (II) Formaldehyd, der bei üblicher destillativer Aufarbeitung langsam entweichen und zur Verunreinigung durch Paraformaldehyd führen würde. Durch die erfindungsgemäße Reaktionsführung wird das weitgehend vermieden.

Zu den einzelnen Reaktionsschritten ist folgendes zu bemerken:
Die Ozonolyse findet z.B. in einem Kolben mit Begasungsrührer statt, das Ozon wird in üblicher Weise zusammen mit Sauerstoff erzeugt und eingeleitet, wobei das Diketen und der Alkohol vorgelegt werden. Die Temperatur des Reaktionsgemisches beträgt bevorzugt -60°C bis+30°C. Man läßt in Abhängigkeit von den Temperaturen so lange reagieren, bis das Diketen verbraucht ist, was durch die Blaufärbung aufgrund des nicht mehr abreagierenden Ozons zu erkennen ist. Die Ozonolyse kann auch bei Raumtemperatur ausgeführt werden, wobei überraschenderweise zwar die Reaktion des Diketens mit Ozon schneller verläuft als die ebenfalls mögliche Reaktion mit dem Alkohol, aber andererseits der Malonsäurehalbester so schnell gebildet wird, daß keine merklichen Zersetzungsprodukte entstehen.

Die folgende Hydrierung, bevorzugt ebenfalls bei -60°C bis +30°C ausgeführt, wird mit den üblichen Hydrierungskatalystoren, wie z.B. Palladium oder Platin auf Aktivkohle oder Raney-Nickel, durchgeführt. Bevorzugt wird die Hydrierung mit H₂/Pd/Aktivkohle ausgeführt.

Auch wenn die Ozonolyse bei tiefen Temperaturen ausgeführt wird, kann die Reaktionsmischung während der Hydrierung auf Raumtemperatur erwärmt werden.

Nach der Hydrierung kann das entstandene Alkylformiat (V) vor oder nach dem Reaktionsschritt c) abgetrennt werden. Das bei einer bevorzugten Ausführungsform des Verfahrens in Methanol entstehende Methylformiat kann z.B. an diesem Punkt des Verfahrens, d.h. vor der Veresterung, abdestilliert werden. Es ist ein wertvolles Ausgangsprodukt beispielsweise für Ameisensäure, Formamid oder Dimethylformamid.

Die anschließende Veresterung des Malonsäurehalbesters (III) unter gleichzeitiger Bildung des Dialkylacetals (V) findet unter den üblichen Bedingungen der säurekatalysierten Veresterung statt. Dazu wird der nach der Hydrierung und der gegebenenfalls erfolgten Abtrennung des Alkylformiats verbliebene Rückstand in einem inerten Lösungsmittel, z.B. Toluol, aufgenommen, mit einer katalytischen Menge Säure, z.B. p-Toluolsulfonsäure, versetzt und erwärmt. Das Reaktionswassr wird azeotrop abdestilliert und die entstandenen Malonsäuredialkylester sowie Dialkylacetale werden bevorzugt destillativ aufgearbeitet. Als Vorlauf im Falle von R-OH = Methanol wird das Dimethylacetal gewonnen. Das Dimethylacetal (Methylal) ist ebenfalls ein interessantes Produkt. Es kann entweder bei der katalytischen Dehydrierung des Methanols zur Herstellung von Formaldehyd eingesetzt werden, wodurch es möglich ist, höhere Formaldehydkonzentrationen zu erzielen, oder es findet Verwendung als stark polares Lösungsmittel mit hoher Alkalibeständigkeit.

Die Kombination der erfindungsgemäßen Verfahrensschritte ergibt ein einfach verlaufendes und hohe Ausbeuten lieferndes Verfahren zur Herstellung von Malonsäuredialkylestern, verbunden mit einer wirtschaftlichen Verwendung der ansonsten unerwünschten Spaltprodukte der Ozonolyse.

### Beispiel

In einem Kolben, der mit einem schnellaufenden Begasungsrührer versehen war, wurden 304 g (3,62 Mol) 98 %iges Diketen und 1,5 l Methanol vorgelegt. Bei einer Temperatur von -40°C wurden 200 l Sauerstoff eingeleitet, der soviel Ozon enthielt, daß 20,5 g Ozon pro Stunde zur Reaktion gebracht wurden. Nach 8 1/4 Stunden trat Blaufärbung ein, die anzeigte, daß das Diketen verbraucht war. Der jodometrisch bestimmte Peroxidgehalt der Lösung betrug nun 97,5 % der Theorie.

Bei -40°C wurden nunmehr 5 g eines Katalysators zugegeben, der 5 % Palladium auf Aktivkohle enthielt. Bei der nun erfolgten Hydrierung stieg die Temperatur im Verlauf von einer Stunde von -40°C auf +10°C an. Insgesamt wurden nur 26,2 l Wasserstoff verbraucht, dies entspricht nur 33 % der zur Hydrierung des Peroxiacetals notwendigen Menge.

Nunmehr wurden 134 g Methylformiat abdestilliert; dies entspricht 92 % der nach dem Wasserstoffverbrauch zu erwartenden Menge.

Zur anschließenden Veresterung wurden 700 ml Toluol sowie 3 g p-Toluolsulfonsäure zugegeben und azeotrop durch Abscheidung des Reaktionswassers entwässert. Hierbei gingen als Vorlauf 81 g Methylal bei 42°C in Form eines Azeotrops mit 9,2 % Methanol über, dies entspricht 89 % der nach dem Wasserstoffverbrauch zu erwartenden Menge.

Nach beendeter Wasserabscheidung destillierte man die Lösungsmittel wie Toluol und Methanol ab und isolierte als letzte Fraktion im Vakuum 420 g Malonsäuredimethylester (Sdp. 45 - 50°C bei 0,1 mbar). Nach dem Gaschromatogramm war dieser 97,5 %ig. Die Ausbeute lag demnach bei 87,5 %, bezogen auf eingesetztes Diketen.

## Patentansprüche

1. Verfahren zur Herstellung von Malonsäuredialkylestern durch Ozonisierung von Diketen, dadurch gekennzeichnet, daß man
a) Diketen in einem C₁-C₆-Alkanol ozonisiert,
b) das Reaktionsgemisch anschließend katalytisch hydriert und
c) dann unter Zugabe katalytischer Säuremengen zur Bildung der Malonsäuredialkylester erwärmt.

2. Verfahren nach Anspruch 1, dadurch gekennzeichnet, daß als Alkohole, C₁-C₄-Alkanole, insbesondere Methanol oder Ethanol, eingesetzt werden.

3. Verfahren nach Anspruch 1, dadurch gekennzeichnet, daß der Reaktionsschritt c) unter den Bedingungen der azeotropen Veresterung erfolgt.

4. Verfahren nach Anspruch 1, dadurch gekennzeichnet, daß man die in Reaktionsschritt b) als Nebenprodukte entstehenden Alkylformiate vor oder nach dem Reaktionsschritt c) abdestilliert.

5. Verfahren nach Anspruch 1, dadurch gekennzeichnet, daß die in Reaktionsschritt c) entstehenden Malonsäuredialkylester und Dialkylacetale destillativ aufgearbeitet werden.

6. Verfahren nach Anspruch 1, dadurch gekennzeichnet, daß die katalytische Hydrierung mit H₂/Pd/Aktivkohle ausgeführt wird.

7. Verfahren nach Anspruch 1, dadurch gekennzeichnet, daß es als Eintopfverfahren durchgeführt wird.

8. Verfahren nach Anspruch 1, dadurch gekennzeichnet, daß die Reaktionsschritte a) und b) bei Temperaturen zwischen -60°C und +30°C ausgeführt werden.

## Claims

1. A process for preparing dialkyl malonates by ozonizing diketene, which comprises
a) ozonizing diketene in a C₁-C₆-alkanol,
b) subsequently catalytically hydrogenating the reaction mixture and
c) then heating, with the addition of catalytic amounts of acid, to form the dialkyl malonates.

2. A process as claimed in claim 1, wherein C₁-C₄-alkanols, especially methanol or ethanol, are employed as alcohols.

3. A process as claimed in claim 1, wherein reaction step c) is carried out under the conditions of azeotropic esterification.

4. A process as claimed in claim 1, wherein the alkyl formates resulting as by-products in reaction step b) are removed by distillation before or after reaction step c).

5. A process as claimed in claim 1, wherein the dialkyl malonates and dialkyl acetals resulting in reaction step c) are worked up by distillation.

6. A process as claimed in claim 1, wherein the catalytic hydrogenation is carried out with H₂/Pd/active carbon.

7. A process as claimed in claim 1, which is carried out as a one-pot process.

8. A process as claimed in claim 1, wherein reaction steps a) and b) are carried out at from -60°C to +30°C.

## Revendications

1. Procédé de préparation de diesters alkyliques d'acide malonique par ozonisation de dicétène, caractérisé en ce que
a) l'on ozonise du dicétène dans un alcanol en C₁-C₆,
b) on soumet ensuite le mélange réactionnel à une hydrogénation catalytique, puis
c) on le chauffe avec addition de quantités catalytiques d'un acide, pour la formation des diesters alkyliques d'acide malonique.

2. Procédé selon la revendication 1, caractérisé en ce qu'on utilise, comme alcools, des alcanols en C₁-C₄, en particulier du méthanol ou de l'éthanol.

3. Procédé selon la revendication 1, caractérise en ce que l'étape réactionnelle c) est exécutée dans les conditions de l'estérification azéotrope.

4. Procédé selon la revendication 1, caractérisé en ce qu'avant ou après l'étape réactionnelle c), on chasse par distillation les formiates d'alkyle formés en tant que produits secondaires dans l'étape réactionnelle b).

5. Procédé selon la revendication 1, caractérisé en ce que les diesters alkyliques d'acide malonique et les dialkylacétals formés dans l'étape réactionnelle c) sont soumis à un traitement ultérieur de distillation.

6. Procédé selon la revendication 1, caractérisé en ce que l'hydrogénation catalytique est effectuée avec H₂/Pd/charbon actif.

7. Procédé selon la revendication 1, caractérisé en ce qu'il est exécuté sous forme de procédé dans un seul et même récipient.

8. Procédé selon la revendication 1, caractérisé en ce que les étapes réactionnelles a) et b) sont conduites à des températures comprises entre -60°C et +30°C.
